# FASCICULE DE BREVET EUROPEEN

(11) **EP 0 715 844 B1**
(45) Date de publication et mention de la délivrance du brevet: **18.12.1996**
(21) Numéro de dépôt: 95402651.4
(22) Date de dépôt: 24.11.1995
(51) Int. Cl.: A61K 7/16

(54) **Compositions à base d'un système abrasif et d'un système tensio-actif**
Präparate mit einem Schleifmittel- und einem Tensid-System
Compositions containing abrasive and detergent system

(30) Priorité: 09.12.1994 FR 9414862
(43) Date de publication de la demande: 12.06.1996
(73) Titulaire: L'OREAL, 75008 Paris (FR)
(72) Inventeur: Ascione, Jean-Marc, F-75018 Paris (FR); Sterle, Pascal, F-95230 Soisy/Montmorency (FR)
(74) Mandataire: Andral, Christophe André Louis

(56) Documents cités:
- FR-A- 2 603 800
- US-A- 4 837 008
- US-A- 5 037 633

## Description

La présente invention concerne de nouvelles compositions comprenant, dans ur support aqueux, un système abrasif contenant un ou plusieurs bicarbonate(s) de métal alcalin, et un système tensio-actif contenant un ou plusieurs tensio-actif(s) non-ionique(s) poly(hydroxypropyléther), ces compositions étant notamment représentées par des compositions pour l'hygiène bucco-dentaire.

L'invention concerne également l'utilisation de ces compositions, comme, ou pour la fabrication d'un, dentifrice sous la forme d'une pâte, d'un gel, d'un liquide ou d'une gomme à mâcher.

Dans le domaine de l'hygiène bucco-dentaire, on utilise couramment des bicarbonates de métal alcalin, notamment pour neutraliser les acides responsables de la carie, et surtout pour leur abrasivité (voir US-A-5 037 633).

De plus, pour formuler les dentifrices dits "aqueux", ainsi qualifiés parce qu'ils contiennent au moins 3% en poids d'eau, on a jusqu'ici utilisé des agents tensio-actifs de type anionique, dont le rôle est d'apporter à la fois un pouvoir moussant et un pouvoir détergent suffisants. Parmi ceux-ci, on peut citer plus particulièrement le lauryl sulfate de sodium, qui est en effet le plus couramment employé.

Or, la demanderesse a observé que dans ces formulations, les bicarbonates de métal alcalin, introduits initialement sous forme de particules solides (poudres), se dissolvaient partiellement, entraînant de ce fait une perte importante du pouvoir abrasif attaché à la composition finale.

Pour pallier à cet inconvénient, la demanderesse a effectué d'importantes recherches qui l'ont amenée à découvrir, de manière fort surprenante, qu'une solubilisation bien moindre du bicarbonate de métal alcalin pouvait être obtenue dans des compositions aqueuses comprenant un système tensio-actif non-ionique du type poly(hydroxypropyléther), par rapport à des compositions aqueuses comprenant un lauryl sulfate de sodium.

Ainsi, à quantités égales de bicarbonates utilisées, le pouvoir abrasif des compositions conformes à l'invention est nettement supérieur à celui des compositions de l'art antérieur.
En outre, le système tensio-actif sélectionné selon l'invention présente un excellent pouvoir moussant et détergent.

Ces découvertes sont à la base de la présente invention.

La présente invention a ainsi pour objet de nouvelles compositions du type comprenant, dans un support aqueux, un système abrasif contenant un ou plusieurs bicarbonate(s) de métal alcalin, et un système tensio-actif, qui sont caractérisées par le fait que ledit système tensio-actif comprend un ou plusieurs tensio-actif(s) non-ionique(s) poly(hydroxypropyléther).

La présente invention a également pour objet des compositions de ce type à usage bucco-dentaire, en particulier des dentifrices.

Elle vise également l'utilisation de ces compositions, comme, ou pour la fabrication d'un, dentifrice qui peut revêtir la forme d'une pâte, d'un gel, d'un liquide ou d'une gomme à mâcher.

Mais d'autres caractéristiques, aspects, objets et avantages de l'invention apparaîtront encore plus clairement à la lecture de la description et des exemples qui suivent.

Le bicarbonate de métal alcalin qui peut être utilisé selon l'invention est de préférence choisi parmi les bicarbonates de sodium ou de potassium.

Les tensio-actifs de type poly(hydroxypropyléther) sont des produits déjà connus en soi.

De préférence, selon la présente invention, le ou les tensio-actifs non-ioniques poly(hydroxypropyléther) sont choisis parmi les composés de structures suivantes:
**(A)** Les composés répondant à la formule **(I)** :

   RO⁅C₃H₅(OH)O]ₙ - H (I)

   dans laquelle le groupement ⁅C₃H₅ (OH)O⁆ représente les structures (I.a), (I.b), et (I.c) suivantes, prises ensemble ou séparément :

   ⁅CH₂-CHOH-CH₂-O⁆ (I.a)

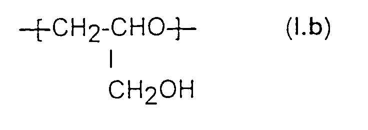

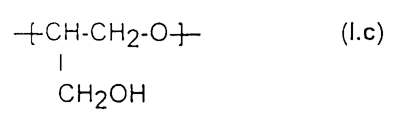

   et R et n ont, ensemble, l'une des signifcations ci-dessous :
   a) R désigne un radical ou un mélange de radicaux alkyles en C₁₀-C_{14 ,} et n est un nombre entier ou décimal variant de 2 à 10 et de préférence de 3 à 6.
   b) R désigne un groupement de formule **(II)**:

      R₂-CO NH-CH₂-CH₂-O-CH₂-CH₂- (II),

      dans laquelle R₂ désigne un radical ou un mélange de radicaux alkyles et/ou alcényles en C₁₁-C₁₇ ,
      et n désigne un nombre entier ou décimal variant de 1 à 5 et de préférence de 1,5 à 4.
   c) R désigne un groupement de formule **(III)** :

      R₃-CHOH-CH₂― (III),

      dans laquelle R₃ désigne un radical aliphatique, cycloaliphatique, arylaliphatique, en C₇-C₂₁, et leurs mélanges, les chaînes aliphatiques désignant en particulier des chaînes alkyles pouvant comporter de 1 à 6 groupements éther, thioéther et/ou hydroxyméthylène,
      et n désigne un nombre entier ou décimal variant de 1 à 10.
      Ces tensio-actifs de formule (I) peuvent être préparés selon les procédés décrits dans les brevets FR 1 477 048, 2 328 763, et 2 091 516.
**(B)** Les composés préparés par condensation, en catalyse acide, et à une température allant de 50 à 120°C, de 2 à 10 et de préférence de 2,5 à 6 moles de glycidol par mole d'alcool ou d'alpha-diol comportant 10 à 14 atomes de carbone, le glycidol étant ajouté lentement à l'alcool ou à l'alpha-diol. Le procédé de préparation de ces composés est décrit dans le brevet FR 2 169 787.
**(C)** Les composés préparés par polyaddition de monochlorhydrine du glycérol sur un composé organique polyhydroxylé en présence d'une base forte, avec élimination au fur et à mesure de l'eau par distillation. Ces composés sont décrits dans le brevet français FR 2 574 786.

Parmi les tensio-actifs non-ioniques poly(hydroxypropyléther) décrits dans les paragraphes (A), (B) et (C) ci-avant, les composés plus particulièrement préférés selon l'invention sont :
i) ceux de formule **(IV)** et **(V)** suivantes : 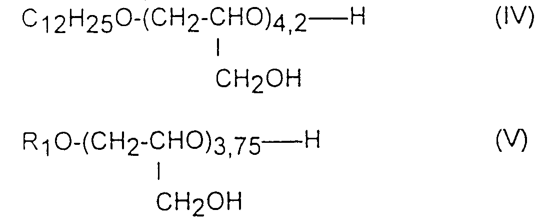 formule (V) dans laquelle R₁ désigne un mélange de radicaux alkyles en C₁₀H₂₁ et C₁₂H₂₅.
2i) les composés préparés par condensation, en catalyse alcaline, de 3,5 moles de glycidol sur un alpha-diol ayant 12 atomes de carbone, selon le procédé décrit dans le brevet FR 2 091 516.
3i) les composés de formule **(VI)** suivante :

   R₂-CONH-CH₂-CH₂-O-CH₂-CH₂-O-(CH₂-CHOH-CH₂-O)_{3,5}―H (VI)

   dans laquelle R₂ désigne un mélange de radicaux comprenant les radicaux alkyles et alcényles suivants: C₁₁H₂₃, C₁₃H₂₇, les radicaux dérivés des acides gras du coprah et le radical dérivé de l'acide oléique.
4i) les composés préparés par condensation de 3,5 moles de glycidol sur un mélange d'alpha-diols en C₁₁-C₁₄, décrits dans le brevet FR 2 091 516.

Le dodécanediol polyglycérolé à 3,5 moles de glycérol est plus particulièrement préféré.

Selon un mode préféré de réalisation des compositions selon l'invention, le système tensio-actif qui leur est attaché n'est constitué que de tensio-actifs non-ionique(s) conforme(s) à l'invention.

Dans les compositions selon l'invention, le ou les bicarbonates de métal alcalin sont généralement présents dans des concentrations pondérales comprises entre environ 0,5 et 80% et de préférence entre environ 1 et 50%, et le ou les tensio-actifs non-ioniques poly(hydroxypropyléther) sont généralement présents dans des concentrations pondérales comprises entre environ 0,1 et 10% et de préférence entre environ 0,2 et 5%, par rapport au poids total de la composition.

Les compositions confomes à l'invention qui sont destinées à l'hygiène bucco-dentaire peuvent contenir, outre le bicarbonate de métal alcalin et le tensio-actif non-ionique poly(hydroxypropyléther), à titre de véhicule ou pour leur activité propre, des excipients ou des ingrédients habituellement utilisés dans les produits à usage bucco-dentaire.

Des compositions conformes à l'invention sont préparées selon les procédés usuels correspondant aux véhicules choisis. Le véhicule physiologiquement acceptable peut être de différente nature selon la forme choisie pour la composition : milieu aqueux ou hydroalcoolique épaissi ou non, excipient pâteux ou solide, gomme, etc...
Selon les formes désirées, ces compositions peuvent également contenir d'autres agents abrasifs, parmi lesquels on peut citer par exemple la silice, l'alumine, le phosphate dicalcique et le carbonate de calcium, des agents anticaries comme par exemple des fluorures de sodium ou de potassium ou d'amine, du monofluorophosphate de sodium, des agents antibactériens tels que par exemple la chlorhexidine, l'alexidine, l'héxétidine, le chlorure de cétyl-pyridinium, le 2,4,4'-trichloro 2'-hydroxydiphényléther, des agents anti-inflammatoires, des agents anti-halitoses, des enzymes, des vitamines, des oligo-éléments, des agents hémostatiques, des agents cicatrisants et des agents actifs sur la gencive.
Elles peuvent contenir par ailleurs d'autres agents usuels tels que des agents de cohésion, des agents édulcorants, humectants ou rafraîchissants, des agents conservateurs, des colorants, des arômes, des agents de sapidité, des agents peptisants et des agents plastifiants.

Des exemples concrets, mais nullement limitatifs, illustrant l'invention vont maintenant être donnés.

### EXEMPLE 1 :

On a étudié, à différentes concentrations, la conservation des cristaux de bicarbonate de sodium dans des solutions aqueuses à 2% de tensio-actif non-ionique poly(hydroxypropyléther)* et la dissolution de ces mêmes cristaux dans des solutions à 2% de lauryl sulfate de sodium, par observation directe desdites solutions au microscope optique.

Pour ce faire, on a réalisé les six compositions ci-dessous (% en poids) qui ont été ensuite portées, sous agitation, à une température de 40°C afin d'obtenir des solutions. On a laissé ensuite revenir ces solutions à la température de 20°C et on les a observées au microscope.

| SOLUTION | NaHCO₃ à 11% | NaHCO₃ à 12% | NaHCO₃ à 13% | Tensio-actif non-ionique* 2% | Lauryl sulfate de Na 2% |
|---|---|---|---|---|---|
| S1 | X | | | X | |
| S2 | X | | | | X |
| S3 | | X | | X | |
| S4 | | X | | | X |
| S5 | | | X | X | |
| S6 | | | X | | X |

| | | | | | |
|---|---|---|---|---|---|
| *Tensio-actif non-ionique poly(hydroxypropyléther) : dodécanediol polyglycérolé à 3,5 moles de glycérol préparé selon le procédé décrit dans le brevet FR 2 091 516. | | | | | |

Les photographies de ces observations sont présentées en annexe.

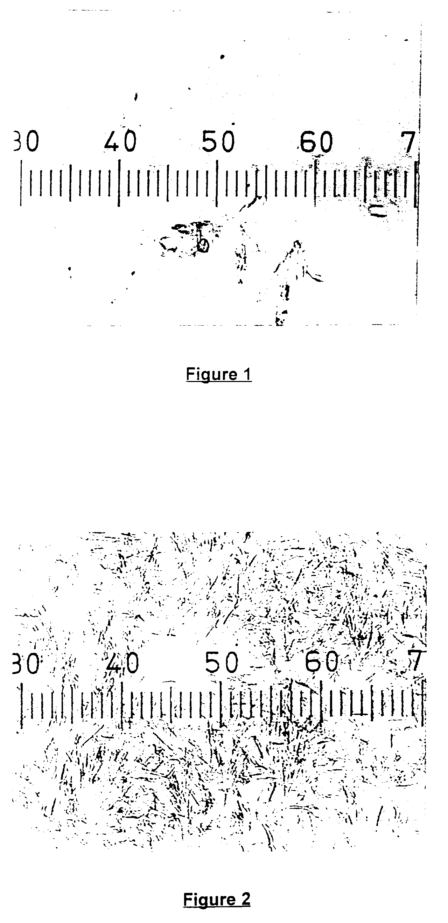

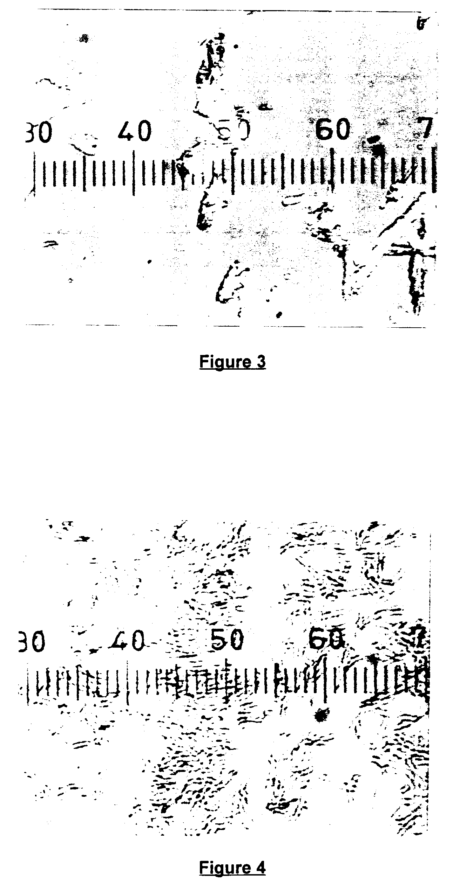

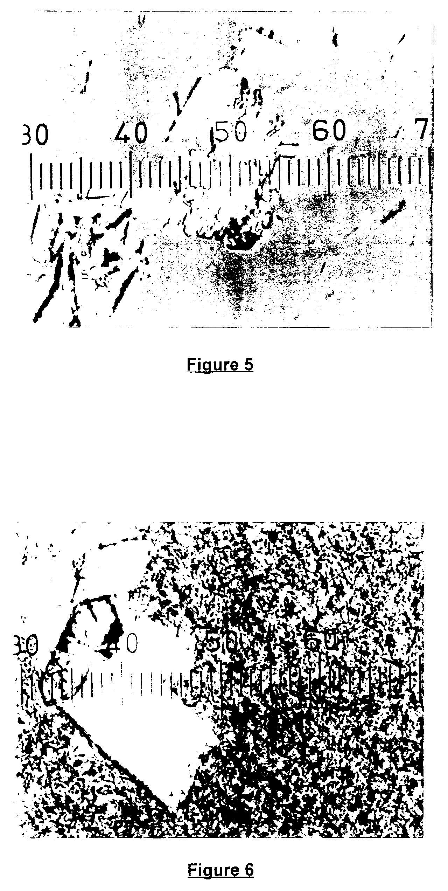

Les figures 1 à 6 correspondent respectivement aux solutions 1 à 6 photographiées.
Dans ces photographies, le réseau cristallin apparaissant en fond des solutions S₂, S₄, et S₆ a été identifié comme étant un réseau de laurylsulfate de sodium.

Les solutions S5 et S6 sont sursaturées en bicarbonate de sodium. Dans les deux cas, on observe donc des cristaux au microscope.
La solution S1 selon l'invention présente des cristaux tandis que la solution S2, qui constitue l'art antérieur, n'en présente plus.
De même, la solution S3 selon l'invention présente des cristaux tandis que la solution S4, qui constitue l'art antérieur, n'en présente plus.

En conclusion, le bicarbonate de sodium se solubilise moins en milieu tensio-actif non-ionique poly(hydroxypropyléther) qu'en milieu lauryl sulfate de sodium.

### EXEMPLE 2 : on illustre ici une pâte dentifrice conforme à l'invention.

| | |
|---|---|
| Bicarbonate de sodium Codex 0/13 vendu par la société SOLVAY | 20g |
| Silice épaississante, vendue sous la dénomination "TIXOSIL 333" par la société RHONE POULENC | 11g |
| Carboxyméthylcellulose de sodium, vendue sous la dénomination "BLANOSE 9M31F" par la société HERCULES | 1,3g |
| Sorbitol en solution aqueuse à 70% de matière active (MA) | 12,6g MA |
| Dodécanediol polyglycérolé à 3,5 moles de glycérol | 1,5g |
| Dioxyde de titane | 0,5g |
| Fluorure de sodium | 0,33g |
| Conservateur, édulcorant, arôme | qs |
| Eau | qsp 100g |

## Revendications

1. Compositions du type comprenant, dans un support aqueux, un système abrasif contenant un ou plusieurs bicarbonate(s) de métal alcalin, et un système tensio-actif, caractérisées par le fait que ledit système tensio-actif comprend un ou plusieurs tensio-actif(s) non-ionique(s) poly(hydroxypropyléther).

2. Compositions selon la revendication 1, caractérisées par le fait que le support aqueux est un véhicule physiologiquement acceptable contenant au moins 3% en poids d'eau par rapport au poids total de la composition.

3. Compositions selon les revendications 1 ou 2, caractérisées par le fait que le ou les bicarbonates de métal alcalin sont choisis parmi le bicarbonate de sodium ou le bicarbonate de potassium.

4. Compositions selon les revendications 1, 2, ou 3, caractérisées par le fait que le ou les tensio-actifs non-ioniques poly(hydroxypropyléther) sont choisis parmi les composés suivants :
**(A)** Les composés répondant à la formule **(I)** :
RO⁅C₃H₅ (OH)O]ₙ - H (I)
dans laquelle le groupement ⁅C₃H₅ (OH)O⁆ représente les structures (I.a), (I.b), et (I.c) suivantes, prises ensemble ou séparément :
⁅CH₂-CHOH-CH₂-O⁆ (I.a) 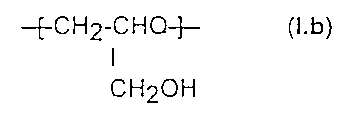 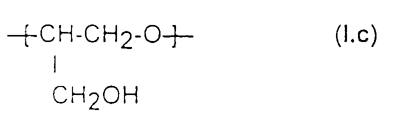
et R et n ont, ensemble, l'une des significations ci-dessous :
a) R désigne un radical ou un mélange de radicaux alkyles en C₁₀-C_{14 ,} et n est un nombre entier ou décimal de 2 à 10 et de préférence de 3 à 6.
b) R désigne un groupement de formule **(II)**:
R₂-CO NH-CH₂-CH₂-O-CH₂-CH₂― (II),
dans laquelle R₂ désigne un radical ou un mélange de radicaux alkyles et/ou alcényles en C₁₁-C₁₇ , et n désigne un nombre entier ou décimal de 1 à 5 et de préférence de 1,5 à 4.
c) R désigne un groupement de formule **(III)** :
R₃-CHOH-CH₂― (III),
dans laquelle R₃ désigne un radical aliphatique, cycloaliphatique, arylaliphatique, en C₇-C₂₁, et leurs mélanges, les chaînes aliphatiques désignant en particulier des chaînes alkyles pouvant comporter de 1 à 6 groupements éther, thioéther et/ou hydroxyméthylène, et n désigne un nombre entier ou décimal de 1 à 10.
**(B)** Les composés préparés par condensation, en catalyse acide, et à une température allant de 50 à 120°C, de 2 à 10 et de préférence de 2,5 à 6 moles de glycidol par mole d'alcool ou d'alpha-diol comportant 10 à 14 atomes de carbone.
**(C)** Les composés préparés par polyaddition de monochlorhydrine du glycérol sur un composé organique polyhydroxylé en présence d'une base forte, avec élimination au fur et à mesure de l'eau par distillation.

5. Compositions selon la revendication 4, caractérisées par le fait que le ou les tensio-actifs non-ioniques poly(hydroxypropyléther) sont choisis parmi les composés suivants :
i) 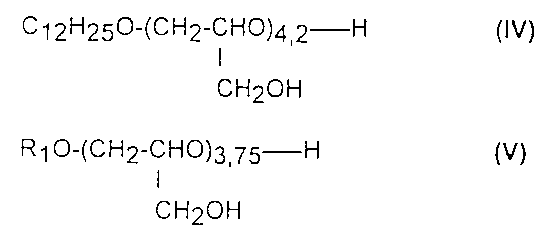 formule (V) dans laquelle R₁ désigne un mélange de radicaux alkyles en C₁₀H₂₁ et C₁₂H₂₅.
2i) les composés préparés par condensation, en catalyse alcaline, de 3,5 moles de glycidol sur un alpha-diol ayant 12 atomes de carbone.
3i) les composés de formule **(VI)** :
R₂-CONH-CH₂-CH₂-O-CH₂-CH₂-O-(CH₂-CHOH-CH₂-O)_{3,5}―H (VI)
dans laquelle R₂ désigne un mélange de radicaux comprenant les radicaux alkyles et alcényles suivants: C₁₁H₂₃, C₁₃H₂₇, les radicaux dérivés des acides gras du coprah et le radical dérivé de l'acide oléique.
4i) les composés préparés par condensation de 3,5 moles de glycidol sur un mélange d'alpha-diols en C₁₁-C₁₄.

6. Compositions selon les revendications 4 ou 5, caractérisées par le fait que le tensio-actif non-ionique poly(hydroxypropyléther) est un dodécanediol polyglycérolé à 3,5 moles de glycérol.

7. Compositions selon l'une quelconque des revendications 1 à 6, caractérisées par le fait qu'il s'agit de compositions pour l'hygiène bucco-dentaire, en particulier de dentifrices.

8. Utilisation des compositions définies à l'une quelconque des revendications précédentes pour la fabrication d'un, dentifrice liquide, d'une pâte dentifrice, d'un gel dentifrice ou d'une gomme à mâcher.

## Claims

1. Compositions of the type comprising, in an aqueous vehicle, an abrasive system containing one or a number of alkali metal bicarbonate(s) and a surfactant system, characterized in that the said surfactant system comprises one or a number of non-ionic poly(hydroxypropyl ether) surfactant(s).

2. Compositions according to Claim 1, characterized in that the aqueous vehicle is a physiologically acceptable vehicle containing at least 3 % by weight of water with respect to the total weight of the composition.

3. Compositions according to Claim 1 or 2, characterized in that the alkali metal bicarbonate(s) are chosen from sodium bicarbonate or potassium bicarbonate.

4. Compositions according to Claim 1, 2 or 3, characterized in that the non-ionic poly(hydroxypropyl ether) surfactant(s) are chosen from the following compounds:
(A) The compounds corresponding to the formula (I):
RO⁅C₃H₅ (OH)O]ₙ - H (I)
in which the ⁅C₃H₅ (OH)O⁆ group represents the following structures (I.a), (I.b) and (I.c), taken together or separately:
⁅CH₂-CHOH-CH₂-O⁆ (I.a) 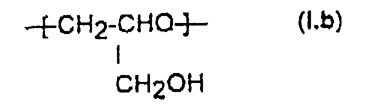 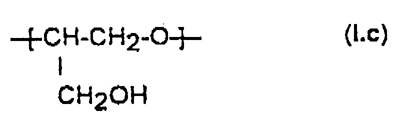
and R and n have, together, one of the meanings below:
a) R denotes a C₁₀-C₁₄ alkyl radical or a mixture of C₁₀-C₁₄ alkyl radicals, and n is a whole or decimal number from 2 to 10 and preferably from 3 to 6,
b) R denotes a group of formula (II):
R₂-CONH-CH₂-CH₂-O-CH₂-CH₂- (II),
in which R₂ denotes a C₁₁-C₁₇ alkyl or alkenyl radical or a mixture of C₁₁-C₁₇ alkyl and/or alkenyl radicals, and n denotes a whole or decimal number from 1 to 5 and preferably from 1.5 to 4,
c) R denotes a group of formula (III):
R₃-CHOH-CH₂- (III),
in which R₃ denotes a C₇-C₂₁ aliphatic, cycloaliphatic or arylaliphatic radical, and their mixtures, the aliphatic chains denoting in particular alkyl chains which can contain from 1 to 6 ether, thioether and/or hydroxymethylene groups,
and n denotes a whole or decimal number from 1 to 10.
(B) The compounds prepared by condensation, in acid catalysis, and at a temperature ranging from 50 to 120°C, of 2 to 10 and preferably of 2.5 to 6 mol of glycidol per mole of alcohol or of alpha-diol containing 10 to 14 carbon atoms.
(C) The compounds prepared by polyaddition of glycerol monochlorohydrin to a polyhydroxylated organic compound in the presence of a strong base, with removal of the water, by distillation, as it is formed.

5. Compositions according to Claim 4, characterized in that the non-ionic poly(hydroxypropyl ether) surfactant(s) are chosen from the following compounds:
i) 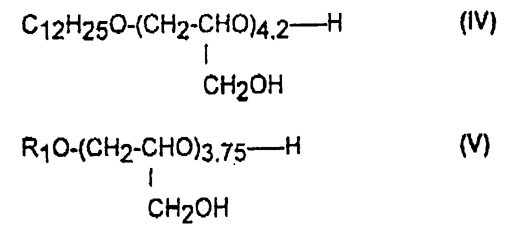 in which formula (V) R₁ denotes a mixture of C₁₀H₂₁ and C₁₃H₂₅ alkyl radicals,
2i) the compounds prepared by condensation, in alkaline catalysis, of 3.5 mol of glycidol with an alpha-diol having 12 carbon atoms,
3i) the compounds of formula (VI):
R₂-CONH-CH₂-CH₂-O-CH₂-CH₂-O-(CH₂-CHOH-CH₂-O)_{3,6}―H (VI)
in which R₂ denotes a mixture of radicals comprising the following alkyl and alkenyl radicals: C₁₁H₂₃, C₁₃H₂₇, the radicals derived from coconut fatty acids and the radical derived from oleic acid,
4i) the compounds prepared by condensation of 3.5 mol of glycidol with a mixture of C₁₁-C₁₄ alpha-diols.

6. Compositions according to Claim 4 or 5, characterized in that the non-ionic poly(hydroxypropyl ether) surfactant is a dodecanediol polyglycerolated with 3.5 mol of glycerol.

7. Compositions according to any one of Claims 1 to 6, characterized in that they are compositions for oral hygiene, in particular dentifrices.

8. Use of the compositions defined in any one of the preceding claims for the manufacture of a liquid dentifrice, a toothpaste, a tooth gel or a chewing gum.

## Patentansprüche

1. Zusammensetzungen, die in einem wäßrigen Träger ein Abrasivmittelsystem, das ein oder mehrere Alkalihydrogencarbonate enthält, und ein Tensidsystem enthalten,
**dadurch gekennzeichnet,** daß das Tensidsystem ein oder mehrere nichtionische grenzflächenaktive Poly(hydroxypropylether) enthält.

2. Zusammensetzungen nach Anspruch 1, dadurch gekennzeichnet, daß der wäßrige Träger ein physiologisch akzeptabler Träger ist, der mindestens 3 Gew.-% Wasser, bezogen auf das Gesamtgewicht der Zusammensetzung, enthält.

3. Zusammensetzungen nach Anspruch 1 oder 2, dadurch gekennzeichnet, daß das oder die Alkalihydrogencarbonate unter Natriumhydrogencarbonat und Kaliumhydrogencarbonat ausgewählt sind.

4. Zusammensetzungen nach einem der Ansprüche 1, 2 oder 3, dadurch gekennzeichnet, daß der oder die nichtionischen grenzflächenaktiven Poly(hydroxypropylether) unter den folgenden Verbindungen ausgewählt sind:
(A) Verbindungen der Formel (I):
RO⁅C₃H₅ (OH)O]ₙ-H (I)
worin die Gruppe ⁅C₃H₅(OH)O⁆ einer oder mehreren der folgenden Strukturen (I.a), (I.b) und (I.c) entspricht:
⁅CH₂-CHOH-CH₂-O⁆ (I.a) 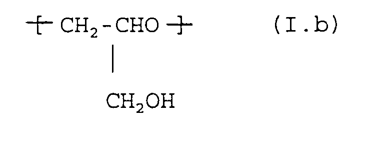 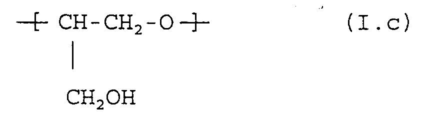
wobei R und n jeweils in Kombination eine der folgenden Bedeutungen haben:
a) R bedeutet eine C₁₀₋₁₄-Alkylgruppe oder ein Gemisch von C₁₀₋₁₄-Alkylgruppen, und n ist eine ganze Zahl oder eine Dezimalzahl im Bereich von 2 bis 10 und vorzugsweise von 3 bis 6;
b) R bedeutet eine Gruppe der Formel (II):
R₂-CO NH-CH₂-CH₂-O-CH₂-CH₂― (II),
worin R₂ eine C₁₁₋₁₇-Alkyl- und/oder eine C₁₁₋₁₇-Alkenylgruppe bedeutet, und n ist eine ganze Zahl oder eine Dezimalzahl im Bereich von 1 bis 5 und vorzugsweise von 1,5 bis 4;
c) R bedeutet eine Gruppe der Formel (III):
R₃-CHOH-CH₂― (III),
worin R₃ eine aliphatische, cycloaliphatische und/oder eine arylaliphatische Gruppe mit 7 bis 21 Kohlenstoffatomen bedeutet, wobei die aliphatischen Gruppen insbesondere Alkylgruppen bezeichnen, die 1 bis 6 Ether-, Thioether- und/oder Hydroxymethylengruppen enthalten können,
und n ist eine ganze Zahl oder eine Dezimalzahl im Bereich von 1 bis 10.
(B) Verbindungen, die unter saurer Katalyse bei einer Temperatur von 50 bis 120 °C durch Kondensation von 2 bis 10 Mol und vorzugsweise 2,5 bis 6 Mol Glycid pro Mol eines Alkohols oder α-Diols mit 10 bis 14 Kohlenstoffatomen hergestellt wurden.
(C) Verbindungen, die durch Polyaddition von Glycerylmonochlorhydrin und einer organischen Polyhydroxyverbindung in Gegenwart einer starken Base hergestellt wurden, wobei das dabei gebildete Wasser durch Destillation entfernt wurde.

5. Zusammensetzungen nach Anspruch 4, dadurch gekennzeichnet, daß der oder die nichtionischen grenzflächenaktiven Poly(hydroxypropylether) unter den folgenden Verbindungen ausgewählt sind:
i) 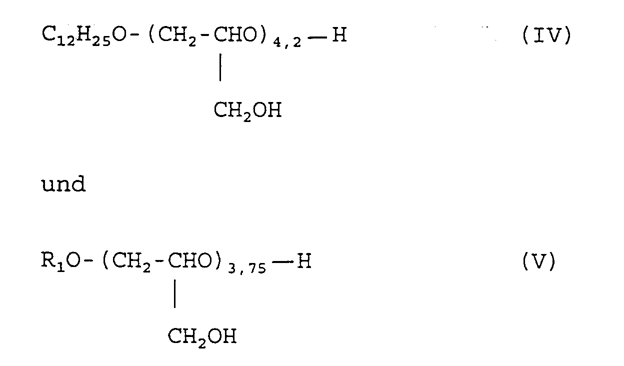 wobei in Formel (V) R₁ ein Gemisch von C₁₀H₂₁- und C₁₂H₂₅-Alkylgruppen bedeutet.
ii) Verbindungen, die unter alkalischer Katalyse durch Kondensation von 3,5 Mol Glycid und einem α-Diol mit 12 Kohlenstoffatomen hergestellt wurden.
iii) Verbindungen der folgenden Formel (VI):
R₂-CONH-CH₂-CH₂-O-CH₂-CH₂-O-(CH₂-CHOH-CH₂-O)_{3,5}―H (VI)
worin R₂ ein Gemisch aus den folgenden Alkyl- und Alkenylgruppen bedeutet: C₁₁H₂₃, C₁₃H₂₇, Gruppen, die von Kopra-Fettsäuren stammen, und der Gruppe, die von Ölsäure abgeleitet ist.
iiii) Verbindungen, die durch Kondensation von 3,5 Mol Glycid und einem Gemisch von C₁₁₋₁₄-α-Diolen hergestellt wurden.

6. Zusammensetzungen nach Anspruch 4 oder 5, dadurch gekennzeichnet, daß der nichtionische grenzflächenaktive Poly(hydroxypropylether), ein mit 3,5 Mol Glycerin verethertes Dodecandiol ist.

7. Zusammensetzungen nach einem der Ansprüche 1 bis 6, dadurch gekennzeichnet, daß es sich um Zusammensetzungen zur Mund- und Zahnpflege und insbesondere um Zahnpasten handelt.

8. Verwendung der Zusammensetzungen nach einem der vorhergehenden Ansprüche als flüssige Zahncreme, Zahnpaste, Zahngel oder Kaugummi oder zu deren Herstellung.
